# DEMANDE DE BREVET EUROPEEN

(11) **EP 4 840 424 A1**
(43) Date de publication de la demande: **18.11.2026**
(21) Numéro de dépôt: 26173825.6
(22) Date de dépôt: 21.04.2026
(51) Int. Cl.: A61F 2/40, A61F 2/46, A61F 2/30

(54) **IMPLANT GLÉNOÏDIEN DE PROTHÈSE D'ÉPAULE**

(30) Priorité: 16.05.2025 FR 2505220
(71) Demandeur: FX Shoulder Solutions, 01440 Viriat (FR)
(72) Inventeur: DAUDET, Jean-Marie, 01440 VIRIAT (FR)
(74) Mandataire: LLR

(57) **Abrégé**

L'invention concerne un ensemble formé par :
- un implant glénoïdien (2) de prothèse d'épaule inversée comprenant :
∘ une embase d'ancrage de l'implant à un os formée par une tige (4) configurée pour s'étendre dans l'épaisseur d'un os (6) depuis une cavité glénoïde, la tige (4) comprenant des orifices traversants (18) et au moins un trou à broche (28), et
∘ des vis de verrouillage (8) de la tige (4) configurées pour traverser radialement la tige (4), par les orifices traversants (18), et l'os (6), lorsque l'implant glénoïdien (2) est implanté dans l'os (6),

- une broche de positionnement (30) de l'implant glénoïdien (2) configurée pour traverser le trou à broche (28) et l'os (6) lorsque l'implant glénoïdien (2) est implanté dans l'os (6) et avant de percer l'os (6) pour mise en place des vis de verrouillage (8).

## Description

### Domaine technique

L'invention concerne un implant glénoïdien, notamment de prothèse de l'épaule inversée, destiné à être fixé à la cavité glénoïdienne d'un patient.

### État de la technique

Une prothèse totale de l'épaule inversée comprend un implant huméral, c'est-à-dire une partie fixée dans l'humérus du patient, et un implant glénoïdien, c'est-à-dire une partie fixée sur la cavité glénoïdienne (aussi appelée glène) du patient. Ces deux implants sont articulés l'un avec l'autre afin de restaurer la mobilité du bras du patient opérée.

Dans le cadre d'une prothèse totale de l'épaule dite « inversée », le centre de rotation de l'articulation est déplacé sur l'implant glénoïdien, à l'inverse d'une prothèse dite « anatomique » où il demeure situé sur l'implant huméral.

Cette modification du positionnement du centre de rotation de l'articulation engendre une modification des muscles sollicités pour mouvoir le bras. En l'espèce, c'est le muscle deltoïde qui est sollicité tandis que pour une prothèse anatomique, les muscles sollicités demeurent ceux formant la coiffe des rotateurs, à savoir les muscles : sous-scapulaire, sus-épineux, sous-épineux et petit rond en plus du deltoïde. Cette modification des muscles impliqués peut s'expliquer par un endommagement irréparable de tout ou partie des muscles de la coiffe des rotateurs, un tel endommagement pouvant intervenir chez les personnes âgées notamment. En d'autres termes, et pour les personnes atteintes d'un endommagement irréparable au niveau des muscles formant la coiffe des rotateurs, une prothèse d'épaule inversée est préférée car elle permet de conserver une mobilité de l'épaule dès les premiers degrés d'abduction du bras par rapport au tronc. La prothèse d'épaule inversée implique donc la sollicitation du muscle deltoïde qui est ancré sur l'humérus de manière plus distale que les muscles de la coiffe des rotateurs, elle compense alors l'endommagement des muscles formant la coiffe des rotateurs.

La prothèse d'épaule inversée est formée par un implant huméral comprenant une extrémité creuse (ou cupule) remplaçant la tête humérale et, au niveau de l'omoplate, une embase d'ancrage osseux (ou métaglène) sur laquelle est rapportée une glénosphère destinée à collaborer avec la cupule positionnée sur l'humérus.

Classiquement, l'embase d'ancrage osseux présent au niveau de l'omoplate est fixée à cet os par l'intermédiaire de plusieurs vis de verrouillage s'étendant à partir de l'embase d'ancrage osseux et divergentes. Cet éloignement permet d'assurer un bon ancrage de l'implant glénoïdien en sollicitant des points de fixation éloignés.

Cependant, les prothèses d'épaule, et plus particulièrement les prothèses d'épaule inversées, sont utilisées pour restaurer l'articulation de personnes souffrant d'arthrose, notamment la prothèse d'épaule inversée pour les raisons de mobilité de l'épaule expliquées ci-dessus. A un stade avancé d'arthrose, la dégradation de l'articulation, et plus particulièrement de la glène de l'omoplate, peut-être trop importante pour envisager un ancrage stable de l'implant glénoïdien classique. En d'autres termes, la surface osseuse disponible ou capital osseux n'est plus suffisante (l'os se creusant), notamment au niveau de la portion reliant la glène à la scapula, pour assurer un ancrage stable de l'embase d'ancrage osseux, ce qui peut remettre en cause la possibilité de restauration de l'articulation par une prothèse d'épaule, même inversée.

Il est dès lors connu de prévoir des vis de verrouillage destinées à traverser l'os et un implant glénoïdien sous forme de tige s'étendant dans l'os de manière à verrouiller l'implant dans l'os et ce même en cas de capital osseux insuffisant pour la mise en place d'une embase d'ancrage.

La mise en place de l'implant implique de plus d'assurer un positionnement précis et une parfaite fixation de cet implant glénoïdien, à défaut de quoi la reproduction de l'articulation naturelle par l'articulation prothétique ne serait pas parfaite. Cela est d'autant plus vrai pour un implant avec des vis de verrouillage tel que décrit ci-dessus : un déplacement, typiquement une rotation, de l'implant entre le moment où l'os est percé pour disposer les vis de verrouillage et celui où les vis de verrouillage sont placées peut conduire à complexifier le placement des vis de verrouillage en cherchant à retrouver un alignement entre les trous ménagés dans l'os et ceux présents sur l'implant glénoïdien.

L'invention a notamment pour but de fournir un ensemble permettant la mise en place d'un implant glénoïdien de prothèse d'épaule inversé stable dans l'omoplate et ce quel que soit le capital osseux d'un patient tout en facilitant le verrouillage de l'implant pour le chirurgien le mettant en place.

### Résumé de l'invention

A cet effet l'invention a pour objet un ensemble formé par :
- un implant glénoïdien de prothèse d'épaule inversée comprenant :
   ∘ une embase d'ancrage de l'implant à un os formée par une tige configurée pour s'étendre dans l'épaisseur de l'os depuis une cavité glénoïde, la tige comprenant des orifices traversants et au moins un trou à broche, et
   ∘ des vis de verrouillage de la tige configurées pour traverser radialement la tige, par les orifices traversants, et l'os, lorsque l'implant glénoïdien est implanté dans l'os,
- une broche de positionnement de l'implant glénoïdien configurée pour traverser le trou à broche et l'os lorsque l'implant glénoïdien est implanté dans l'os et avant de percer l'os pour mise en place des vis de verrouillage.

Ainsi, et au lieu d'utiliser une embase d'ancrage difficile à stabiliser, la tige s'étend dans l'épaisseur de l'os, dans une travée osseuse, et est stabilisée dans l'os par des vis de verrouillage traversant radialement la tige et l'os. On assure donc un ancrage stable de l'implant glénoïdien avec une structure s'étendant moins (une tige) en exploitant des zones osseuses moins atteintes, ce qui est particulièrement adapté aux personnes souffrant d'arthrose à un stade avancé.

De plus, l'utilisation d'une broche de positionnement permet une stabilisation transitoire de la tige dans l'os pendant le verrouillage de cette dernière à l'aide des vis de verrouillage. On évite donc tout risque de déplacement, en rotation ou en translation, pendant le verrouillage de la tige dans l'os pour faciliter le travail du chirurgien.

Suivant d'autres caractéristiques optionnelles de l'ensemble prises seules ou en combinaison :
- L'implant glénoïdien comprend une glénosphère rapportée sur la tige,
- la tige comprend une face de réception d'une entretoise de fixation de la tige à la glénosphère,
- la face de réception comprend un alésage configuré pour collaborer avec l'entretoise et une gorge entourant l'alésage, la gorge étant configurée pour interagir avec un outil de préhension et de guidage de la tige,
- l'entretoise est formée d'une première portion de fixation de l'entretoise à la tige et d'une seconde portion de fixation de l'entretoise à la glénosphère, la première portion et la seconde portion étant décalées l'une par rapport à l'autre,
- la première portion et la seconde portion sont inclinées l'une par rapport à l'autre,
- l'implant glénoïdien comprend en outre une pièce stabilisatrice de l'implant glénoïdien par rapport à l'os lorsque l'implant glénoïdien est implanté dans l'os,
- la pièce stabilisatrice comprend deux extrémités de stabilisation configurées pour s'étendre de part et d'autre de l'os lorsque l'implant glénoïdien est implanté dans l'os.
- L'ensemble comprend en outre un outil de préhension et de guidage de l'implant glénoïdien, comprenant une extrémité de verrouillage à la tige et une extrémité de préhension, l'extrémité de préhension s'étendant au moins en partie parallèlement à la tige lorsque l'outil de préhension et de guidage est fixé à la tige, l'extrémité de préhension comprenant :
   ∘ au moins un trou de guidage pour la mise en place des vis de verrouillage, et
   ∘ au moins un trou de guidage pour la mise en place de la broche de positionnement,
- l'extrémité de préhension est mobile en rotation par rapport à l'extrémité de verrouillage,
- l'ensemble comprend un premier élément de guidage d'un positionnement de la broche de positionnement configuré pour être disposé entre l'extrémité de préhension et l'os de manière à réaliser un perçage de l'os pour la mise en place de la broche de positionnement,
- l'ensemble comprend un deuxième élément de guidage d'un positionnement de la broche de positionnement configuré pour être disposé entre l'extrémité de préhension et l'os de manière à disposer la broche de positionnement,
- l'ensemble comprend un troisième élément de guidage d'un positionnement des vis de verrouillage configuré pour être disposé entre l'extrémité de préhension et l'os de manière à réaliser un perçage de l'os pour la mise en place des vis de verrouillage, et
- un diamètre du deuxième élément de guidage d'un positionnement de la broche de positionnement est inférieur à un diamètre du premier élément de guidage d'un positionnement de la broche de positionnement.

### Brève description des figures

L'invention sera mieux comprise à la lecture de la description qui va suivre donnée uniquement à titre d'exemple et faite en se référant aux dessins annexés dans lesquels :
La [Fig. 1] est une vue en perspective d'un implant glénoïdien de prothèse d'épaule inversée d'un ensemble selon l'invention,
La [Fig. 2] est une vue en perspective d'une tige d'un implant glénoïdien de prothèse d'épaule inversée d'un ensemble selon l'invention,
La [Fig. 3] est une vue en perspective d'un outil de préhension et de guidage de l'implant glénoïdien et d'une broche de positionnement selon l'invention placé sur une omoplate,
La [Fig. 4] est une vue en perspective d'un outil de préhension et de guidage de l'implant glénoïdien et d'une broche de positionnement selon l'invention placé sur une omoplate,
La [Fig. 5] est une vue en perspective d'un outil de préhension et de guidage de l'implant glénoïdien et d'une broche de positionnement selon l'invention placé sur une omoplate avec un foret à pointer,
La [Fig. 6] est une vue en perspective d'un outil de préhension et de guidage de l'implant glénoïdien et d'une broche de positionnement selon l'invention placé sur une omoplate avec un guide de perçage mesureur, et
La [Fig. 7] est une en perspective d'un outil de préhension et de guidage de l'implant glénoïdien et d'une broche de positionnement selon l'invention placé sur une omoplate avec des moyens de vissage.

### Description détaillée

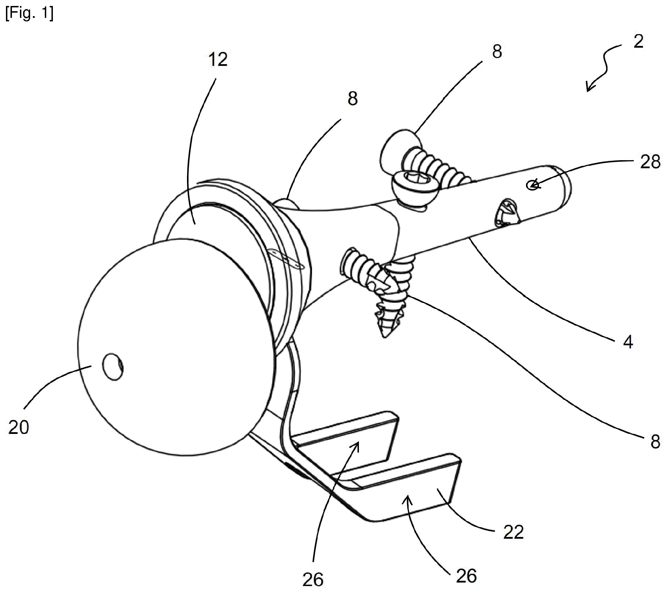

On se réfère désormais à la figure 1 illustrant un implant glénoïdien 2 de prothèse d'épaule inversée.

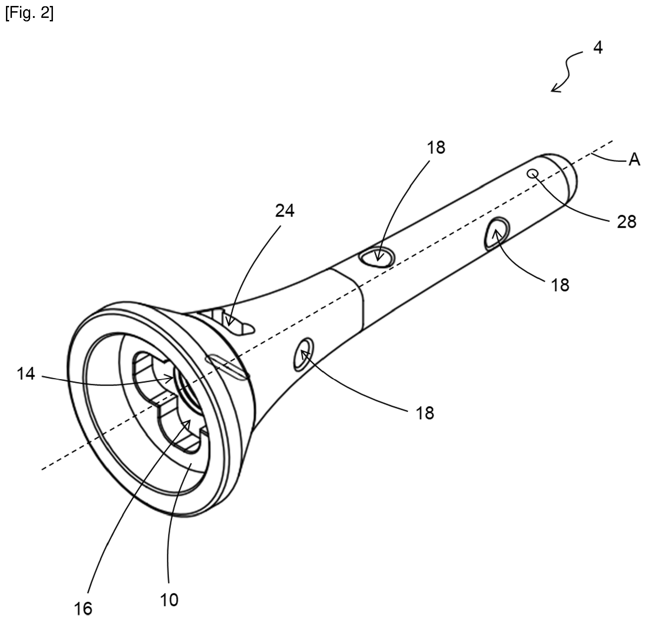

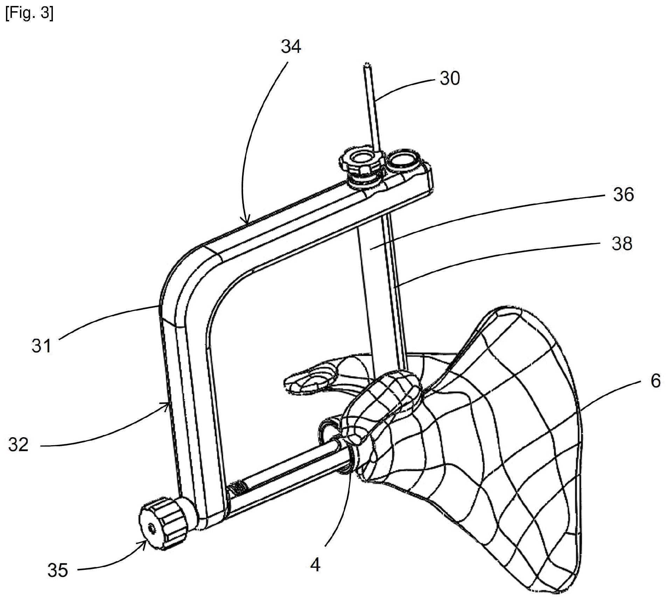

Cet implant glénoïdien 2 comprend une embase d'ancrage formée par une tige 4 configurée pour s'étendre dans l'épaisseur d'un os 6 (une omoplate visible sur les figures 3à 7) depuis une cavité glénoïde et par des vis de verrouillage 8 (ou vis corticales) de la tige 4 dans l'os configurées pour traverser radialement la tige 4 et l'os 6. La tige 4 s'étend dans une travée osseuse de l'os 6, par exemple au niveau de la jonction entre la scapula et l'acromion, pour permettre un ancrage, par mise en place des vis de verrouillage 8, sans trop s'étendre dans l'os 6, ce qui pourrait poser problème en cas d'arthrose à un stade trop avancé. On comprend donc que le chirurgien va procéder à la réalisation d'un alésage dans l'épaisseur de l'os 6 pour y loger la tige 4. Les vis de verrouillage 8 sont ensuite mises en place pour traverser radialement l'os 6 et la tige 4 (par des orifices traversants 18 de cette dernière) et ainsi verrouiller la tige 4 dans l'os 6. On comprend bien évidemment que « radialement » signifie selon une direction perpendiculaire à un axe longitudinal A de la tige 4 (illustré sur la figure 2).

La tige 4, visible seule sur la figure 2, est réalisée dans un matériau biocompatible et ayant une durée de vie optimale la plus longue possible. Elle peut par exemple être réalisée en alliage de titane, en acier inoxydable, en chrome cobalt ou encore à partir d'un matériau polymère. La longueur de la tige 4 peut varier afin de s'adapter au mieux à la taille de l'os 6, avec un diamètre pouvant varier entre 5 et 14 millimètres. Il est possible à cet effet de concevoir plusieurs tiges 4 de tailles variables et longueur variable ou une tige 4 formée par un assemblage de plusieurs parties (chaque partie peut par exemple portée au moins un orifice traversant 18) dont les longueurs peuvent varier afin d'assembler une tige 4 adaptée à l'os 6. La longueur de la tige 4 peut par exemple varier entre 40 et 120 millimètres.

La tige 4 peut comprendre une face de réception 10 d'une entretoise 12, la face de réception 10 pouvant comprendre un alésage 14, par exemple taraudé, permettant la fixation de l'entretoise 12 via une vis et ou un cône ou un clip, par exemple par vissage si l'alésage 14 est taraudé, potentiellement complété par une gorge 16 formant une surface d'interaction avec un outil de préhension et de guidage décrit plus bas.

La tige 4 comprend en outre des orifices traversants 18 permettant le passage des vis de verrouillage 8, ici au nombre de trois. De préférence, les orifices traversants 18 sont réalisés au niveau des zones de la tige 4 qui seront localisées au niveau des zones de l'os 6 où l'épaisseur de ce dernier sera la plus importante. Ainsi, les vis de verrouillage 8 traverseront une épaisseur d'os 6 la plus importante possible. Il est par ailleurs possible de verrouiller la tige 4 en sollicitant l'acromion et la coracoïde.

Comme entrevu ci-dessus, la tige 4 peut recevoir une entretoise (ou bague rapportée) 12. Cette dernière permet d'assurer une liaison à distance entre la tige 4 et une glénosphère 20.

l'entretoise 12 peut être formée d'une première portion de fixation de l'entretoise 12 à la tige 4 et d'une seconde portion de fixation de l'entretoise 12 à la glénosphère 20, la première portion et la seconde portion étant décalées l'une par rapport à l'autre. Les portions peuvent être des pièces indépendantes ou des parties d'une seule et même pièce. Les deux portions peuvent alternativement être alignées sans aucun décalage.

La première portion et la seconde portion peuvent être ou non inclinées l'une par rapport à l'autre.

Cette entretoise 12 peut être formée d'un seul tenant ou par plusieurs parties assemblées l'une à l'autre. Elle peut être totalement coaxiale de la tige 4 ou présenter un axe de fixation de la glénosphère 20 décalé par rapport à l'axe de fixation de l'entretoise 12 sur la tige 4. Ces axes de fixation peuvent être parallèles entre eux ou non. Ces différentes configurations permettent d'adapter l'implant glénoïdien 2 au patient la recevant. L'entretoise 12 est rapportée sur la tige 4 après que cette dernière ait été verrouillée sur l'os 6.

L'entretoise 12 et la glénosphère 20 ne feront pas l'objet d'une description détaillée dans la présente demande de brevet.

L'implant glénoïdien 2 peut en outre comprendre une pièce stabilisatrice 22 (ou fourchette stabilisatrice). Cette dernière, visible sur la figure 1, peut être rapportée sur la tige 4, qui comprend dès lors une fente de fixation 24 de la pièce stabilisatrice 22. Plus précisément, la fente de fixation 24 peut déboucher au niveau d'un taraudage interne de la tige 4. Une pièce intermédiaire comprenant un filetage externe peut dès lors être vissée à la tige 4 et verrouiller la pièce stabilisatrice 22.

Cette pièce stabilisatrice 22 peut comprendre une extrémité de verrouillage, avantageusement percée, de la pièce stabilisatrice 22 à la tige 4 et deux extrémités de stabilisation 26. Ces dernières sont disposées en regard d'une de l'autre de sorte à s'étendre de part et d'autre de l'os 6 (ici de la scapula) lorsque l'implant glénoïdien 2 est mis en place. On peut envisager qu'au moins une extrémité de stabilisation 22 soit en appui contre l'os 6 lorsque l'implant glénoïdien 2 est mis en place. Elle peut dans ce cas avoir une surface plane s'étendant parallèlement à l'os 6 afin d'obtenir un appui sur une surface importante. Le nombre et la forme des extrémités de stabilisation 22 peut varier.

La pièce stabilisatrice 22 pourrait également permettre un ancrage osseux supplémentaire pour l'implant glénoïdien 2. Au moins une extrémité de stabilisation 26 (ou l'extrémité de stabilisation s'il n'y en a qu'une) peut dans ce cas comprendre un orifice traversant pour la fixer à l'os 6, par exemple par vissage et ainsi créer un point d'ancrage supplémentaire.

La pièce stabilisatrice 22 peut être réalisée dans un matériau biocompatible, ayant une durée de vie optimale la plus longue possible et permettant une bonne reprise des efforts. Elle peut par exemple être réalisée en alliage de titane, en acier inoxydable, en chrome cobalt ou encore à partir d'un matériau polymère.

La tige 4 comprend également un trou de broche 28 destinée à être traversé par une broche de positionnement 30 (visible sur les figures 3 à 7). La broche de positionnement 30 est configurée pour traverser le trou à broche 28 et l'os 6 lorsque l'implant glénoïdien 2 est implanté sur l'os 6 et avant de percer l'os 6 pour mise en place des vis de verrouillage 8. Comme expliqué plus haut, cela permet de stabiliser provisoirement la tige 4 pendant le perçage de l'os pour le placement des vis de verrouillage 8 et la fixation par ces dernières. On évite ainsi tout mouvement de la tige 4, en rotation et/ou en translation, dans l'os 6 pendant le processus de mise en place des vis de verrouillage 8, ce qui facilite cette mise en place.

La mise en place de la broche de positionnement 30 nécessite un perçage de l'os 6 passant par le trou de broche 28 pour ensuite placer la broche de positionnement 30 qui va donc traverser l'os - et la tige 4 en passant au travers du trou de broche 28.

Les figures 3 à 7 illustrent quant à elles la tige 4 placé dans l'os 6 et attachée à un outil de préhension et de guidage 31. Ce dernier comprend une extrémité de verrouillage 32 configurée pour collaborer avec la gorge 16 et l'alésage 14 afin d'attacher la tige 4 à l'outil de préhension et de guidage 31 et de pouvoir placer cette dernière dans la cavité ménagée dans l'os 6. L'outil de préhension et de guidage 31 comprend également une extrémité de préhension 34 de l'ensemble formé par ce dernier et la tige 4 par un chirurgien.

L'extrémité de verrouillage 32 et l'extrémité de préhension 34 sont avantageusement deux parties séparées de l'outil de préhension et de guidage 31. Dès lors, l'extrémité de verrouillage 32 est dans un premier temps rapportée sur la tige 4 (que cette dernière soit munie ou non de la pièce stabilisatrice 22). Cette extrémité de verrouillage 32 est avantageusement creuse et partiellement taraudée de manière à y insérer une pièce intermédiaire comprenant au moins un filetage externe de manière à verrouiller par vissage l'extrémité de verrouillage 32 sur la tige 4.

L'extrémité de préhension 34 est ensuite rapportée sur l'extrémité de verrouillage 32 et fixée à cette dernière, par exemple en les vissant l'une à l'autre, par exemple par une vis à tête moletée 35.

L'extrémité de préhension 34 comprend une partie s'étendant parallèlement à la tige 4 lorsque cette dernière est tenue par l'outil de préhension et de guidage 31 et comprend des trous pouvant être alignés avec les orifices traversants 18. Ces trous de guidage permettent de guider un chirurgien lors de la mise en place de la tige 4. En effet, l'alignement lui permet de savoir où percer l'os 6 pour retrouver un orifice traversant 18. Il peut pour cela positionner un élément de guidage 36 (aussi appelé « troisième élément de guidage 36 »), ici de forme cylindrique, s'étendant à partir des trous de guidage et perpendiculairement à la tige 4 et à la partie de l'extrémité de préhension 34 portant les trous de guidage 46. Cet élément de guidage 36 vient dès lors en butée contre l'os 6 et est avantageusement creux de manière à pouvoir y introduire des moyens de perçage de l'os 6 puis les vis de verrouillage 8 et des moyens de vissage de ces dernières à la tige 4. Les vis de verrouillage 8 traversent dès lors l'os 6 et la tige 4.

Avant cela, la broche de positionnement 30 est placée au niveau de l'os 6. Pour ce faire, ce dernier est percé de manière à former un trou passant par le trou de broche 28 et traversant l'os 6 de part en part. L'extrémité de préhension 34 joue le même rôle que pour la mise en place d'une vis de verrouillage 8 en comprenant au moins un trou de guidage pour la mise en place de la broche de positionnement 30.

Plus précisément, il est possible de disposer un premier élément de guidage d'un positionnement de la broche de positionnement 30 entre l'extrémité de préhension 34 (qui comprend un orifice traversant donnant accès au premier élément de guidage ainsi qu'au deuxième décrit ci-après) et l'os 6 de manière à réaliser un perçage de l'os 6 pour la mise en place de la broche de positionnement 30.

Il est également possible de disposer un deuxième élément de guidage 38 d'un positionnement de la broche de positionnement 30 entre l'extrémité de préhension 34 et l'os 6 de manière à disposer la broche de positionnement 30 dans le trou réaliser dans l'os 6.

Il est possible que le diamètre du deuxième élément de guidage 38 du positionnement de la broche de positionnement 30 soit inférieur au diamètre du premier élément de guidage du positionnement de la broche de positionnement 30. Dans une telle configuration, il est possible de placer le deuxième élément de guidage 38 sans avoir à retirer le premier et ainsi éviter toute déstabilisation de l'ensemble. Alternativement, il est possible de retirer le premier élément de guidage avant placement du deuxième élément de guidage.

Les figures 3 à 7 illustrent différentes configurations de l'ensemble selon l'invention.

Dans la figure 3, la broche de positionnement 30 est déjà en place (elle traverse l'os 6 et le trou de broche 28) Elle traverse un élément de guidage, par exemple le deuxième élément de guidage 38. L'outil de préhension et de guidage 31 est donc en place, la broche de positionnement 30 traverse l'extrémité de préhension 34. Dans une telle configuration, la broche de positionnement 30 bloque l'outil de préhension et de guidage 31 en rotation et en translation par rapport à l'os 6, ce qui assure un blocage de la tige 4 verrouillée sur l'extrémité de verrouillage 32.

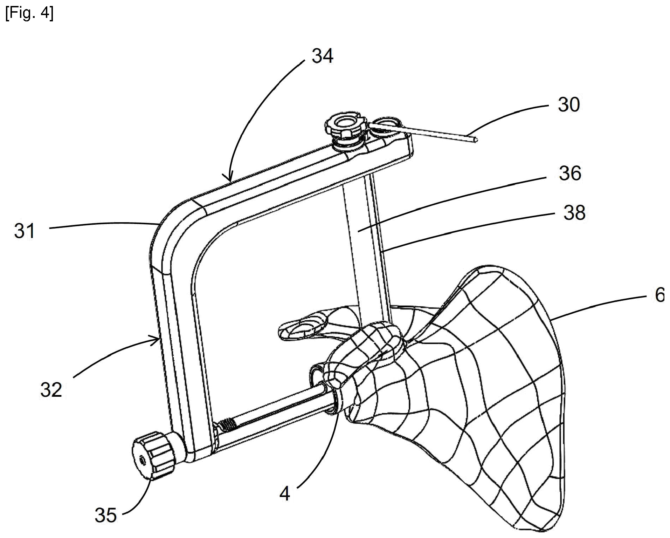

La figure 4 illustre une possibilité de cintrage de la broche de positionnement 30 pour éviter une gêne du chirurgien.

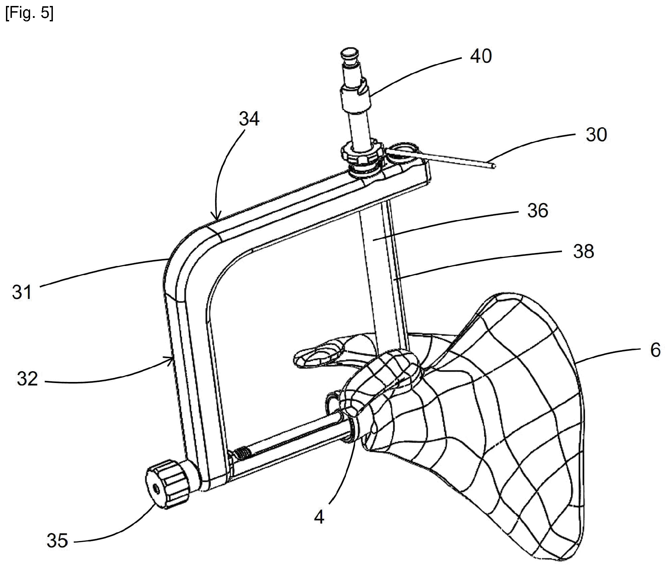

La figure 5 illustre la mise en place d'un moyen de perçage, par exemple un foret à pointer 40, dans le troisième élément de guidage 36 afin de percer l'os 6 pour réaliser un trou passant par un orifice traversant pour la mise en place d'une vis de verrouillage 8.

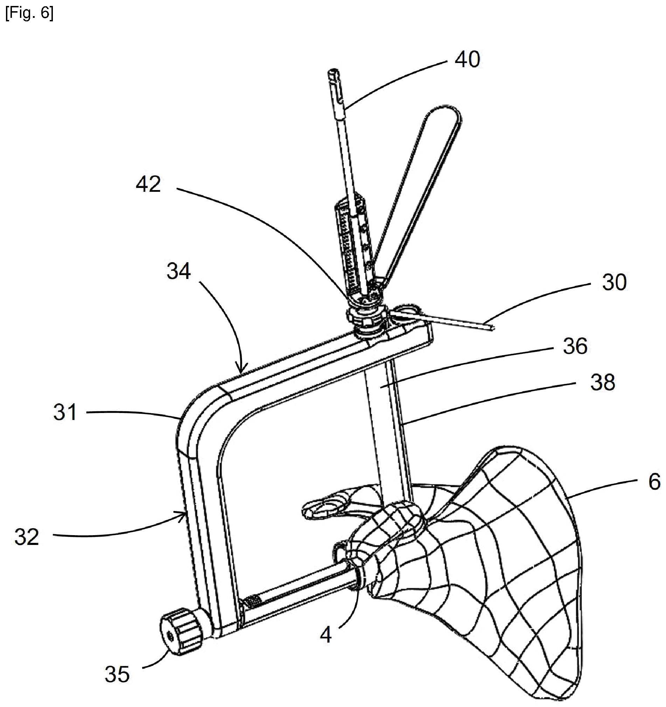

La figure 6 illustre la mise en place de moyens de mesure d'une profondeur de perçage, par exemple un guide de perçage mesureur 42. Il est dès lors possible de percer l'os 6, par exemple avec prise de mesure d'une profondeur de perçage.

Les moyens utilisés pour le perçage de l'os 6 peuvent être retirés. La broche de positionnement 30 est toujours en position de manière à stabiliser l'ensemble.

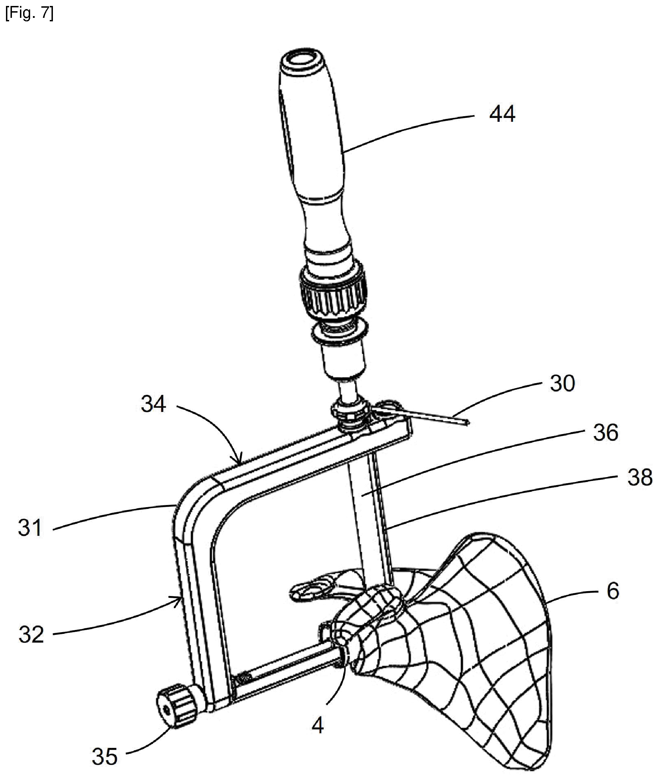

Il est dès lors possible, comme illustré sur la figure 7, d'introduire une vis de verrouillage 8 dans le troisième élément de guidage 36 et de moyens de vissage 44, manuels ou automatisés, afin de visser la vis de verrouillage 8 dans la tige 4 au travers de l'os 6.

Les opérations permettant la mise en place d'une vis de verrouillage 8 peuvent être réitérées de manière à placer plusieurs vis de verrouillage 8, par exemple par rotation de l'extrémité de préhension 34 et avec utilisation d'autres orifices réalisés au niveau de cette dernière.

Il est ensuite possible de retirer la broche de positionnement 30 (car la tige 4 est verrouillée dans l'os 6 par les vis de verrouillage 8) et de désolidariser l'extrémité de verrouillage 32, et donc l'outil de préhension et de guidage 31, de la tige afin de finaliser le montage de l'implant glénoïdien 2 sur l'os 6.

L'invention n'est pas limitée aux modes de réalisation présentés et d'autres modes de réalisation apparaîtront clairement à la personne du métier.

### Liste de références

2 : implant glénoïdien
4 : tige
6 : os
8 : vis de verrouillage
10 : face de réception
12 : entretoise
14 : alésage
16 : gorge
18 : orifices traversants
20 : glénosphère
22 : pièce stabilisatrices
24 : fente de fixation
26 : extrémités de stabilisation
28 : trou de broche
30 : broche de positionnement
31 : outil de préhension et de guidage
32 : extrémité de verrouillage
34 : extrémité de préhension
35 : vis à tête moletée
36 : troisième élément de guidage
38 : deuxième élément de guidage
40 : foret à pointer
42 : guide de perçage mesureur
44 : moyens de vissage
A : axe longitudinal

## Revendications

1. Ensemble formé par :
- un implant glénoïdien (2) de prothèse d'épaule inversée comprenant :
∘ une embase d'ancrage de l'implant à un os formée par une tige (4) configurée pour s'étendre dans l'épaisseur d'un os (6) depuis une cavité glénoïde, la tige (4) comprenant des orifices traversants (18) et au moins un trou à broche (28), et
∘ des vis de verrouillage (8) de la tige (4) configurées pour traverser radialement la tige (4), par les orifices traversants (18), et l'os (6), lorsque l'implant glénoïdien (2) est implanté dans l'os (6),
- une broche de positionnement (30) de l'implant glénoïdien (2) configurée pour traverser le trou à broche (28) et l'os (6) lorsque l'implant glénoïdien (2) est implanté dans l'os (6) et avant de percer l'os (6) pour mise en place des vis de verrouillage (8).

2. Ensemble selon la revendication 1, dans lequel la tige (4) comprend une face de réception (10) d'une entretoise (12) de fixation de la tige (4) à une glénosphère (20).

3. Ensemble selon l'une quelconque des revendications précédentes, dans lequel l'implant glénoïdien (2) comprend en outre une pièce stabilisatrice (22) de l'implant glénoïdien (2) par rapport à l'os (6) lorsque l'implant glénoïdien (2) est implanté sur l'os (6).

4. Ensemble selon l'une quelconque des revendications précédentes, l'ensemble comprenant en outre un outil de préhension et de guidage (31) de l'implant glénoïdien (2), comprenant une extrémité de verrouillage (32) à la tige (4) et une extrémité de préhension (34), l'extrémité de préhension (34) s'étendant au moins en partie parallèlement à la tige (4) lorsque l'outil de préhension et de guidage (31) est fixé à la tige (4), l'extrémité de préhension (34) comprenant :
- au moins un trou de guidage pour la mise en place des vis de verrouillage (8), et
- au moins un trou de guidage pour la mise en place de la broche de positionnement (30).

5. Ensemble selon la revendication 4, dans lequel l'extrémité de préhension (34) est mobile en rotation par rapport à l'extrémité de verrouillage (32).

6. Ensemble selon la revendication 2 combinée aux revendications 4 ou 5, dans lequel la face de réception (10) comprend un alésage (14) configuré pour collaborer avec l'entretoise (12) et une gorge (16) entourant l'alésage (14), la gorge (14) étant configurée pour interagir avec l'outil de préhension et de guidage (31) de la tige (4).

7. Ensemble selon l'une quelconque des revendications 4 à 6, comprenant un premier élément de guidage d'un positionnement de la broche de positionnement (30) configuré pour être disposé entre l'extrémité de préhension (34) et l'os (6) de manière à réaliser un perçage de l'os (6) pour la mise en place de la broche de positionnement (30).

8. Ensemble selon l'une quelconque des revendications 4 à 7, comprenant un deuxième élément de guidage (38) d'un positionnement de la broche de positionnement (30) configuré pour être disposé entre l'extrémité de préhension (34) et l'os (6) de manière à disposer la broche de positionnement (30) dans l'os (6).

9. Ensemble selon l'une quelconque des revendications 7 ou 8, dans lequel un diamètre du deuxième élément de guidage (38) d'un positionnement de la broche de positionnement (30) est inférieur à un diamètre du premier élément de guidage d'un positionnement de la broche de positionnement (30).

10. Ensemble selon l'une quelconque des revendications 4 à 9, comprenant un troisième élément de guidage (36) d'un positionnement de vis de verrouillage (8) configuré pour être disposé entre l'extrémité de préhension (34) et l'os (6) de manière à réaliser un perçage de l'os (6) pour la mise en place des vis de verrouillage (8).
